# EUROPEAN PATENT SPECIFICATION

(11) **EP 1 030 841 B1**
(45) Date of publication and mention of the grant of the patent: **22.09.2004**
(21) Application number: 98955389.6
(22) Date of filing: 13.11.1998
(51) Int. Cl.: C07D 211/90, A61K 31/44

(54) **PROCESS FOR THE PREPARATION OF 1,4-DIHYDROPYRIDINES AND COMPOUNDS USED IN THIS PROCESS**
VERFAHREN ZUR HERSTELLUNG VON 1,4-DIHYDROPYRIDINE SOWIE IN DIESEM VERFAHREN VERWENDETE VERBINDUNGEN
PROCEDE DE PREPARATION DE 1,4-DIHYDROPYRIDINES ET COMPOSES UTILISES AU COURS DE CE PROCEDE

(30) Priority: 14.11.1997 DK 129997
(43) Date of publication of application: 30.08.2000
(73) Proprietor: A/S GEA Farmaceutisk Fabrik, 2650 Hvidovre (DK)
(72) Inventor: KARUP, Gunnar, Leo, DK-2300 Copenhagen S (DK); PREIKSCHAT, Herbert, Fritz, DK-3460 Birkerod (DK); PEDERSEN, Soren, Bols, DK-2650 Hvidovre (DK)
(74) Representative: Bagger-Soerensen, Birgitte
(86) International application number: PCT/DK1998/000492
(87) International publication number: WO 1999/025688

(56) References cited:
- EP-A- 0 089 167
- EP-A- 0 225 175
- EP-B- 0 177 965
- CA-A- 2 188 071
- J.E. ARROWSMITH ET AL.: "Long acting dihydropyridine calcium antagonists" JOURNAL OF MEDICINAL CHEMISTRY., vol. 29, no. 9, 1986, pages 1696-1702, XP002070765 WASHINGTON US cited in the application
- HOBSON PUBLISHING: 'Dihydropyridines in Action', 1989
- ROBERTS; CASEIRO: 'Basic Principles of Organic Chemistry', 1964
- MARCH: 'Advanced Organic Chemistry, 4th Ed', 1992
- MARCH: 'Advanced Organic Chemistry, 2nd Ed', 1992
- STREITWIESER; HEATHCOCK: 'Introduction to Organic Chemistry', 1981

## Description

The present invention relates to a process for the preparation of certain 1,4-dihydropyridines having an amino group attached to a substituent in the 2-position of the 1,4-dihydropyridinium ring and pharmaceutically acceptable acid addition salts thereof. In addition, the invention relates to novel intermediates of use for such purpose.

Compounds belonging to this class of 1,4-dihydropyridines have shown activity as calcium-channel blockers and have found utility as anti-ischaemic and antihypertensive agents. Furthermore, compounds of this class have been used in the treatment of Raynaud's syndrome.

A particularly preferred compound of this class of 1,4-dihydropyridines is the compound, 2-(2-aminoethoxymethyl)-4-(2-chlorophenyl)-6-methyl-1,4-dihydropyridine-3,5-dicarboxylic acid 3-ethyl ester 5-methyl ester, known under the generic name, amlodipine.

EP 0 089 167 B discloses certain 1,4-dihydropyridines having an amino-containing group attached to the 2-position, i.a. amlodipine, and their preparation.

According to said patent, the 1,4-dihydropyridines are prepared by removal of the amino-protecting group from the corresponding amino-protected 1,4-dihydropyridine or by reduction of the corresponding azido compound into the amine. As well the amino-protected 1,4-dihydropyridines as the azido compounds are prepared by the well-known Hantzsch synthesis.

In Example 11 of the patent an overall yield of 1 %, only, is stated for the production of amlodipine maleate starting with the reaction of 2-azido ethanol with ethyl 4-chloroacetoacetate, whereas an overall yield of 8.8 % is disclosed in a later publication by the inventors of EP 0 089 167 B, J. Med. Chem., (1986), 29, 1696 - 1702, see particularly pp. 1700 - 1701.

The overall yield for the alternative process has not been given in the patent and cannot be calculated on the basis of the information given therein. However, in part calculated on the basis of yields reported by others having reproduced the process, the overall yield seems to be in the order of 12 - 20 %, starting with the reaction of 2-phthalimido ethanol with ethyl 4-chloroacetoacetate and ending with removal of the protecting group and preparation of the maleate salt.

The present invention provides a process whereby the 1,4-dihydropyridines can be obtained in higher overall yield. Furthermore, the use of potentially explosive azide starting materials (see e.g. Chem. Ind., (1986), 10, 337) is avoided.

By the process according to the invention, the 1,4-dihydropyridines are prepared starting from an acetal intermediate which is reacted with hydroxylamine or a derivative thereof so as to produce an oxime intermediate which is reduced to provide the desired 1,4-dihydropyridine, optionally as a pharmaceutically acceptable acid addition salt thereof. Hereby the 1,4-dihydropyridines can be obtained in excellent yields. E.g. amlodipine, maleate has been obtained in a yield of about 62 % calculated on the acetal intermediate.

Furthermore, the acetal intermediate in itself can be obtained in excellent yield by the Hantzsch synthesis, as described in the following. Thus, the amlodipine acetal intermediate has been obtained in a yield of 42 % starting with the reaction of 2,2-diethoxyethanol with ethyl 4-chloroacetoacetate, and accordingly an overall yield of amlodipine, maleate of 29 %, calculated on the 2,2-diethoxyethanol, has been obtained.

CA 2,188,071 A discloses a process for the preparation of 1,4-dihydropyridine derivatives, i.a. amlodipine, by reductive amination of the corresponding aldehyde using ammonium acetate and sodium cyanohydride in a protic solvent such as methanol, or reaction of the aldehyde with hydroxylamine hydrochloride and base to give the corresponding oxime followed by reduction with ammonium formate in methanol in the presence of palladium hydroxide on charcoal.

In CA 2,188,071 A it is stated that the dihydropyridine derivatives are formed in good yields employing easily available precursors, and that the overall yield is far greater than the prior art, i.e. 46 % for amlodipine. This yield, however, seems to be calculated on the compound, 4-(2-chlorophenyl)-2-(2,3-dihydroxypropoxymethyl)-1,4-dihydro-6-methyl-3,5-pyridinedicarboxylic acid 3-ethyl 5-methyl ester, in CA 2,188,071 A designated IC. But as this compound already includes the 1,4-dihydropyridine ring, an "overall" yield calculated on this basis cannot be compared to the overall yields indicated above.

A calculation on a comparable basis, i.e. based on the compound being reacted with ethyl 4-chloroacetoacetate, viz. the compound 2,2-dimethyl-[1,3]dioxolane-4,5-dimethanol, gives a yield of the aldehyde intermediate of about 15 % resulting in an overall yield of amlodipine of about 7 %, i.e. several times smaller than the overall yield of about 29 % which has been obtained via the acetal intermediate used as starting material in the process according to the invention.

Also the yields, which have been obtained by conversion of the aldehyde intermediate into amlodipine by the process according to CA 2,188,071 A (about 48 % by the reductive amination and about 43 % by conversion via the oxime), are far below the yield of about 62 %, which has been obtained by the process according to the invention using the acetal intermediate as starting material, which yield even includes the preparation of the maleate salt.

Incidentally, CA 2,188,071 A discloses an acetal, viz. the compound 4-(2-chlorophenyl)-2-(2,2-dimethoxyethoxymethyl)-1,4-dihydro-6-methyl-3,5-pyridinedicarboxylic acid 3-ethyl 5-methyl ester, as well as its preparation from 2,2-dimethoxyethanol and 2-(2-chloromethyl)-4-(2-chlorophenyl)-1,4-dihydro-6-methyl-3,5-pyridinedicarboxylic acid 3-ethyl 5-methyl ester and its conversion into a bicyclic structure forming an oxazine ring with the pyridine nitrogen atom (cf. Examples 11 and 12). However, neither its conversion into the corresponding oxime nor its preparation by Hantzsch synthesis has been disclosed. There is no mentioning either of any possible use of the compound as intermediate in the preparation of amlodipine.

CA 2,188,071 A also includes a general formula XX for an acetal, but the formula includes two undefined substituents, R₁₀ and R₁₁, and accordingly it cannot be considered an anticipating disclosure of any specific acetal. Furthermore, neither the conversion of the acetal into the corresponding oxime nor its preparation by Hantzsch synthesis has been disclosed. There is no mentioning either of any possible use of the acetal as intermediate in the preparation of amlodipine or any other 1,4-dihydropyridine having a substituent with an amino group in the 2-position of the 1,4-dihydropyridinium ring.

EP 225 175 A2 discloses a substantive number of 1,4-dihydropyridine derivatives and different processes for their preparation. Amlodipine is not among the disclosed derivatives. One of the disclosed processes is a process for the preparation of a 1,4-dihydropyridine derivative, which like the compounds prepared by the process according to the invention, has a (2-aminoethoxy)methyl substituent in the 2-position, but differs from said compounds in having a fluoromethyl substituent in the 6-position. The 1,4-dihydropyridine derivative is prepared by reduction of the corresponding oxime which in turn is prepared by reaction of the corresponding acetal with hydroxylamine.

However, EP 225 175 A2 does not mention anything about the acetal being obtained directly by the Hantzsch synthesis and even less the particular advantages obtainable thereby.

On the contrary, the acetal is prepared from the corresponding bromomethyl substituted 1,4-dihydropyridine derivative obtained by reaction of the corresponding methyl substituted 1,4-dihydropyridine derivative with pyridinium perbromide, a process which would be unsuitable for the preparation of the acetal used in the process according to the present invention due to side reactions.

Thus, it can be concluded, that the use of the acetal intermediate in the preparation of the particular 1,4-dihydropyridines being prepared by the process according to the present invention presents substantive advantages over the prior art and cannot be considered obvious in view thereof.

Accordingly, the invention provides an inventive process for the preparation of 1,4-dihydropyridines of the general formula I 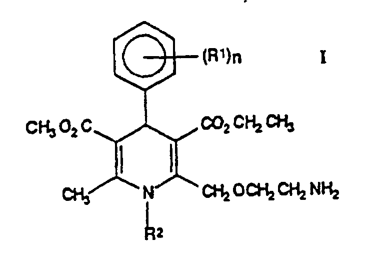 wherein
R¹ each, independently, represents H, Cl or CF₃,
R² represents H, C₁-C₅ alkyl, C₃-C₆ cycloalkyl or aralkyl, and
n is 1 or 2,
or acid addition salts thereof,
comprising the steps of reacting an acetal of the general formula II 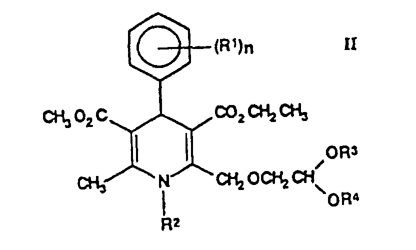 wherein
R¹, R² and n have the same meanings as defined above, and
R³ and R⁴, which may be the same or different, represent C₁-C₅ alkyl, C₃-C₆ cycloalkyl, aralkyl or together represent -(CH₂)ₘ-, wherein
m is 2 or 3,
with

R⁵ONH₂

or an acid addition salt thereof, wherein
R⁵ represents H, C₁-C₅ alkyl, C₃-C₆ cycloalkyl or aralkyl,
so as to provide an oxime of the general formula III 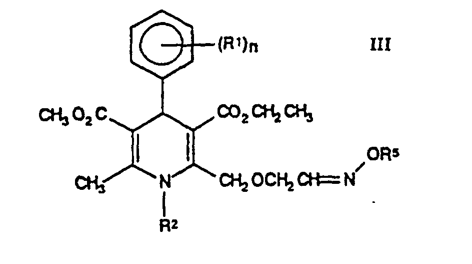 wherein
R¹, R², R⁵ and n have the same meanings as defined above, and
reducing the formed oxime of formula III so as to provide a 1,4-dihydropyridine of formula I, and, if desired, converting a compound of formula I obtained as the free base into a pharmaceutically acceptable acid addition salt thereof or vice versa.

The reaction of the acetal of formula II with R⁵ONH₂ or an acid addition salt thereof to give the oxime of formula III is carried out in an appropriate solvent, such as an alcoholic solvent comprising a lower alkanol, such as methanol, ethanol or isopropanol, e.g. in admixture with water. In a presently preferred embodiment an aqueous solution of R⁵ONH₂, hydrochloride is combined with a solution of the acetal in methanol, and the mixture is heated to reflux for a suitable period, such as 2 - 8 hours, normally around 4 hours.

The reduction of the oxime of formula III into the desired 1,4-dihydropyridine of formula I is carried out using a suitable reduction agent selected from the numerous reduction agents being known for the reduction of oximes into amines, see e.g. the surveys given in R. C. Larock, "Comprehensive Organic Transformations", VCH Publishers, (1989). p. 424, Houben-Weyl: "Methoden der Organischen Chemie", Vol. E16d, Part 2, (1992), pp. 884 - 893, and Houben-Weyl: "Methoden der Organischen Chemie", Vol. XI/1, (1957), pp. 495 -504.

According to a particular embodiment of the invention, the reduction is carried out by catalytical hydrogenation, preferably using a nobel metal catalyst, such as platinum or palladium, or a Raney nickel catalyst. The reduction is preferably carried out under acidic conditions.

In a presently preferred embodiment, the reduction is carried out by catalytical hydrogenation in acetic acid using palladium-on-carbon as a catalyst.

According to another, although less preferred embodiment of the invention, the reduction is carried out using sodium borohydride/nickel chloride hydrate as reduction agent.

As other examples of catalysts, which may be of use for the present purpose, the following can be mentioned: sodium borohydride in combination with other compounds, such as titanium tetrachloride or molybdenum trichloride; lithium aluminum hydride or zinc powder.

A compound of formula I obtained as the free base may, if desired, be converted into a pharmaceutically acceptable acid addition salt thereof or vice versa. The hydrochloride, hydrobromide, sulphate, phosphate or acid phosphate, acetate, maleate, fumarate, besylate, lactate, tartrate, citrate and gluconate salts are examples of such pharmaceutically acceptable acid addition salts. The maleate and the besylate salts are particularly preferred.

With the exception of the compound 4-(2-chlorophenyl)-2-(2,2-dimethoxy-ethoxymethyl)-1,4-dihydro-6-methyl-3,5-pyridine-dicarboxylic acid 3-ethyl 5-methyl ester, the acetales of formula II are novel compounds and as such represent a particular aspect of the invention.

A specific group of acetales according to the invention, are the compounds of the general formula II 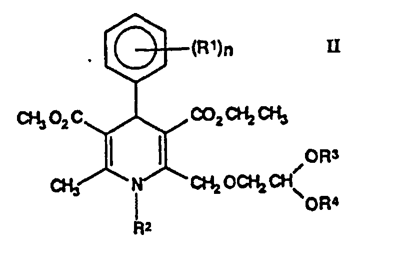 wherein
R¹ each, independently, represents H, Cl or CF₃,
R² represents H, C₁-C₅ alkyl, C₃-C₆ cycloalkyl or aralkyl,
R³ and R⁴, which may be the same or different, represent C₁-C₅ alkyl, C₃-C₆ cycloalkyl, aralkyl or together represent -(CH₂)ₘ-, wherein
m is 2 or 3, and
n is 1 or 2,
with the proviso that when R² is H, and no R¹ is CF₃, then R³ and R⁴ are other than methyl.

A preferred group of acetales of the general formula II is represented by the compounds wherein n is 1, R¹ is chloro in the 2-position of the phenyl ring, R² is H, and R³ and R⁴, which may be the same or different, represent C₂-C₅ alkyl.

Particularly preferred is the compound 4-(2-chlorophenyl)-2-(2,2-diethoxy-ethoxymethyl)-6-methyl-1,4-dihydro-pyridine-3,5-dicarboxylic acid 3-ethyl ester 5-methyl ester.

The acetals of formula II can be obtained directly by the Hantzsch synthesis and have been obtained in excellent yield by this synthesis.

Accordingly, in the process according to the invention the acetal of formula II is obtained by a Hantzsch synthesis carried out using a compound containing a group of the formula 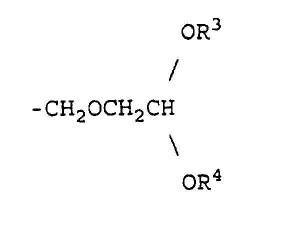 wherein
R³ and R⁴ have the same meanings as defined above, as one of the reactants in the Hantzsch condensation.

In principle the Hantzsch synthesis is carried out by reacting an aldehyde with a β-keto ester and an aminocrotonic acid ester as illustrated in the following Scheme 1:

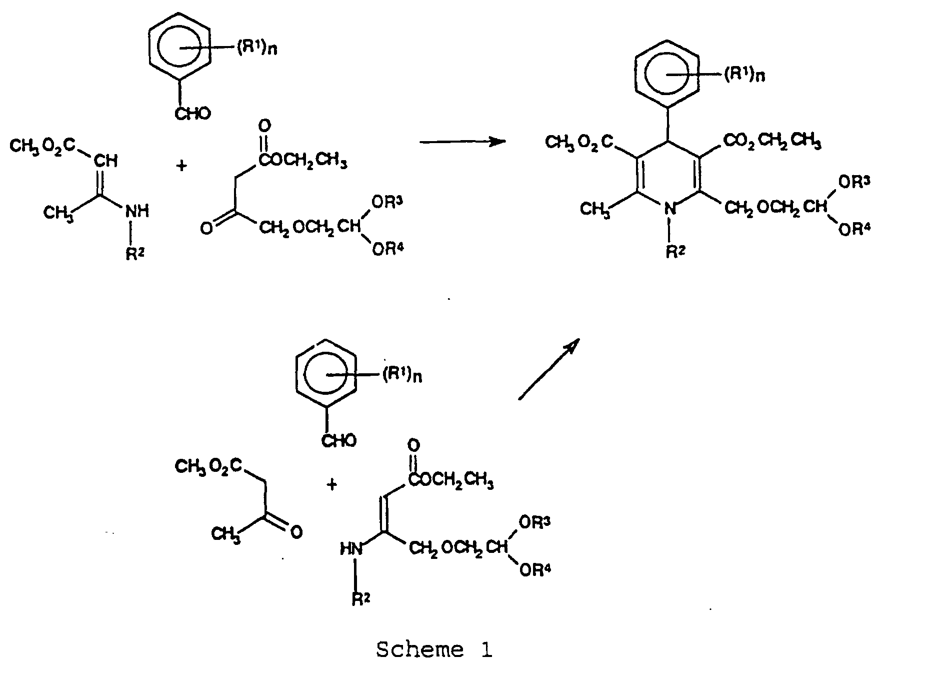

However, as is known in the art, various modifications of the Hantzsch synthesis are possible.

Instead of performing the reaction in one step as illustrated above, the synthesis can be carried out using preformed intermediates, e.g. as illustrated in the following Schemes 2, 3 and 4:

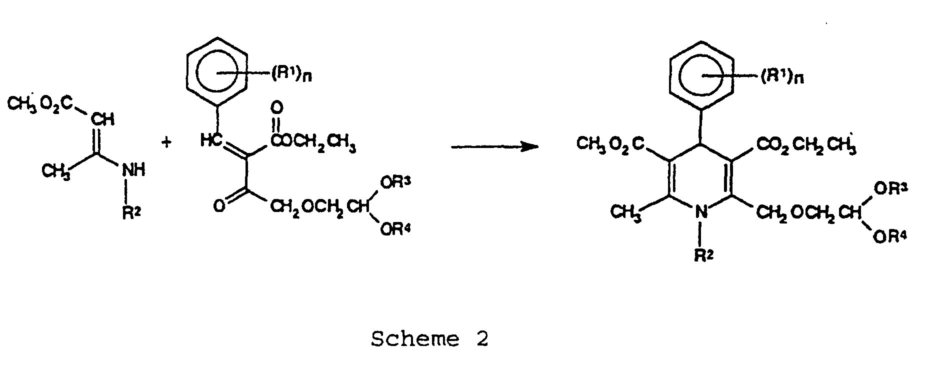

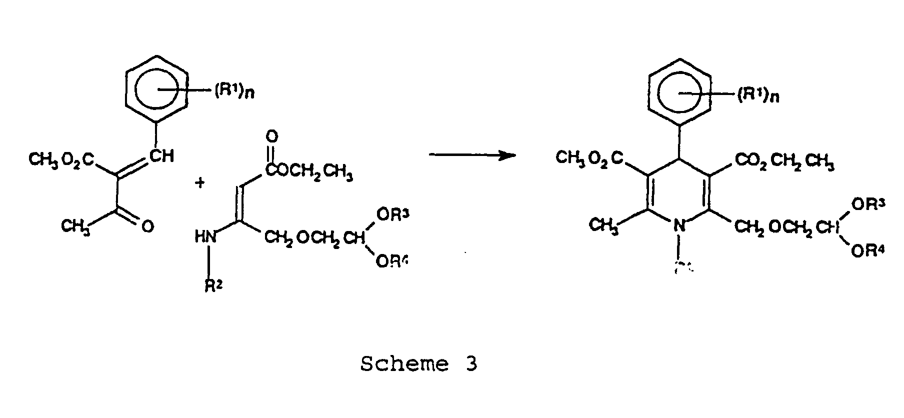

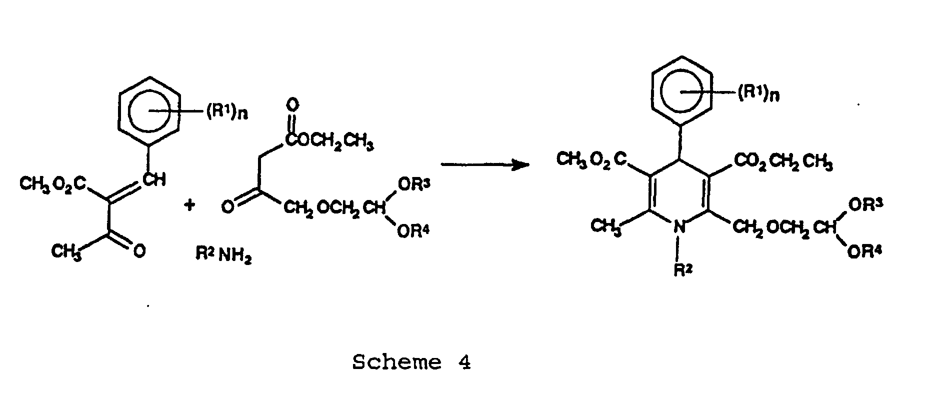

As will be appreciated by a person skilled in the art the above schemes are only examples and other modifications of the Hantzsch synthesis can be made without deviating from the scope and spirit of the invention.

In a further preferred embodiment of the process according to the invention, the Hantzsch synthesis, or at least one step thereof, is carried out in a solvent being capable of forming an azeotrope with water, particularly toluene, benzene or xylene. Hereby, water of reaction can be removed as an azeotrope with the solvent during the reaction.

Some of the oximes of formula III are novel compounds.

The only oximes of formula III being specifically disclosed in CA 2,188,071 A are the compound, 4-(2-chloro-phenyl)-2-(2-hydroxyimino-ethoxymethyl)-6-methyl-1,4-dihydro-pyridine-3,5-dicarboxylic acid 3-ethyl ester 5-methyl ester and the corresponding 2-methoxyimino compound.

Accordingly, the invention also provides the oximes of the general formula III 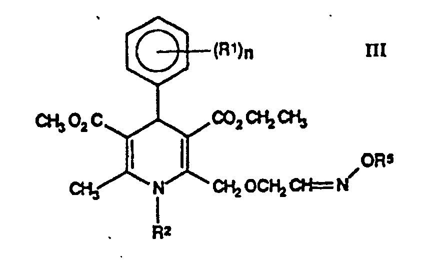 wherein
R¹ each, independently, represent H, Cl or CF₃,
R² represents H, C₁-C₅ alkyl, C₃-C₆ cycloalkyl or aralkyl,
R⁵ represents H, C₁-C₅ alkyl, C₃-C₆ cycloalkyl or aralkyl, and
n is 1 or 2,
with the exception of the compounds 4-(2-chlorophenyl)-2-(2-hydroxyimino-ethoxymethyl)-6-methyl-1,4-dihydro-pyridine-3,5-dicarboxylic acid 3-ethyl ester 5-methyl ester and 4- (2-chloro-phenyl)-2-(2-methoxyiminoethoxymethyl)-6-methyl-1,4-dihydro-pyridine-3,5-dicarboxylic acid 3-ethyl ester 5-methyl ester.

A particular group of compounds of formula III are the compounds wherein
R¹ each, independently, represent H, Cl or CF₃,
R² represents H, C₁-C₅ alkyl, C₃-C₆ cycloalkyl or aralkyl,
R⁵ represents H, C₁-C₅ alkyl, C₃-C₆ cycloalkyl or aralkyl, and
n is 1 or 2,
with the proviso that when R² is H and no R¹ is CF₃, then R⁵ is C₃-C₆ cycloalkyl or aralkyl.

In the present specification and claims, the definition C₁-C₅ alkyl includes linear and branched alkyl groups like methyl, ethyl, propyl, incl. n-propyl and i-propyl, butyl, incl. n-butyl, sec.-butyl and tert.-butyl, and pentyl, incl. n-pentyl and tert.-pentyl. The definition C₃-C₆ cycloalkyl includes cyclopropyl, cyclobutyl, cyclopentyl and cyclohexyl, and the definition aralkyl includes groups having a phenyl or naphthyl group, particularly a phenyl group, as the aryl moiety and a C₁-C₅ alkyl group as defined above as the alkyl moiety, the benzyl group being a particularly preferred aralkyl group.

The invention will now be further illustrated by specific examples which, however, should not be regarded as any limitation of the scope of the invention.

### EXAMPLES.

### Preparation of starting materials.

### Example A. 4-(2,2-Diethoxy-ethoxy)-3-oxo-butyric acid ethyl ester (1).

To a stirred suspension of 58,8 g 60 % (1.47 mol) sodium hydride in 600 ml anhydrous tetrahydrofuran, a solution of 94 g (0.7 mol) 2,2-diethoxy-ethanol in 160 ml tetrahydrofuran was added dropwise, so that the temperature was kept below 40 'C. After completion of the addition, the reaction mixture was stirred for further 30 minutes. Then 115 g (0.7 mol) ethyl 4-chloroacetoacetate in 500 ml anhydrous tetrahydrofuran was added dropwise within 3 hours, so that the temperature was kept between 10 °C and 40 °C, preferentially at about 20 'C. The mixture was stirred overnight at room temperature. Then 90 ml ethanol was added dropwise, and the mixture was poured into 900 g of ice after which pH was adjusted to 6 with hydrochloric acid. The organic phase was separated and dried over MgSO₄. The tetrahydrofuran was evaporated off and the product was separated from the oily layer in a separation funnel. Then the product was dissolved in toluene and purified by filtration through a short column of silica. The toluene was evaporated off, leaving the product as a light yellow oil. The product was purified by distillation in vacuo.
yield: 130.9 g = 71.4 %
bp. = 112-114 'C at 0.2 mm Hg

| Elemental analysis: | | |
|---|---|---|
| Calculated | C 54.9% | H 8.5% |
| Found | C 54.48% | H 8.7% |

IR: 2986 cm⁻¹; 1726 cm⁻¹; 1748 cm⁻¹; 1119 cm⁻¹; 1067 cm⁻¹ (Between KBr plates)
NMR: 250 MHz 1H-NMR (CDCl₃) (δ ppm):
4.646 (t, H, CH); 4.286 (s, 2H, CH2); 4.224 (s, 2H, CH2); 3,708 (q, 2H, CH2); 3.568 (q, 2H, CH2); 3.556 (d, 2H, CH2) ; 1.30 (t, 3H, CH3); 1.21 (m, 6H, CH3).

### Example B. 3-(2-chloro-phenyl)-2-[2-(2,2-diethoxy-ethoxy)-acetyl]-acrylic acid ethyl ester (cis and trans isomer) (2).

A solution of 53 g (0.38 mol) 2-chlorobenzaldehyde, 98 g (0.38 mol) 4-(2,2-diethoxy-ethoxy)-3-oxo-butyric acid ethyl ester (1) and 6 ml piperidine in 1600 ml toluene was refluxed in a Dean-Stark water separator for four hours until 6.2 ml of water had been separated (theoretical amount = 6.5 ml). The reaction mixture was cooled to room temperature and washed twice with 200 ml of water, then with 500 ml of a saturated solution of sodium bisulphite and finally with 200 ml of water. The mixture was dried over MgSO₄ and the toluene was evaporated off to give the product as a dark red oil.
yield: 99.3 g = 67.9 %

| Elemental analysis: | | | |
|---|---|---|---|
| Calculated | C 59.3% | H 6.5% | Cl 9.2% |
| Found | C 59.41% | H 7.11% | Cl 9.2% |

IR: 2977 cm⁻¹; 2931 cm⁻¹; 1725 cm⁻¹; 1617 cm⁻¹;1443 cm⁻¹; 1375 cm⁻¹; 1253 cm⁻¹; 1121 cm⁻¹; 1057 cm⁻¹; 760 cm⁻¹; (Between KBr plates)
NMR: 500 Mhz 1H-NMR (CDCl₃) (δ ppm):
Mixture of geometric isomers
8.07, 7.97 (2 s cis,trans, 1H, CH); 7.48-7.12 (m, 4H, ArH); 4.656, 4.576 (2 t cis,trans, 2H, CH); 4.3 (d.d., 2H, CH2); 4.154 (m, 2H, CH2); 3.682 (m, 2H, CH2); 3.53 (m, 4H, CH2); 1.324 (t, 3H, CH3); 1.184 (t, 6H, CH3).

### Example C. 3-amino-4-(2,2-diethoxy-ethoxy)but-2-enoic acid ethyl ester (3).

A mixture of 26.2 g (0.1 mol) 4-(2,2-diethoxyethoxy)-3-oxo-butyric acid ethyl ester **(1)** and 8.47 g (0.11 mol) of ammonium acetate in 75 ml of ethanol was refluxed for 60 minutes. The ethanol was evaporated off and the resulting crude 3-amino-4-(2,2-diethoxy-ethoxy)but-2-enoic acid ethyl ester was dissolved in 100 ml of toluene and washed twice with 75 ml of water. The organic phase was evaporated off and the crude product was distilled in vacuo giving the pure compound as a colourless liquid.
bp. = 130-131 °C at 0.3 mm Hg
yield: 23.0 g = 88 %

| Elemental analysis: | | | |
|---|---|---|---|
| Calculated | C 55.2% | H 8.9% | Cl 5.4% |
| Found | C 55.91% | H 8.9% | Cl 5.4% |

IR: 3445 cm⁻¹; 3336 cm⁻¹; 2976 cm⁻¹; 2930 cm⁻¹; 1669 cm⁻¹; 1622 cm⁻¹; 1564 cm⁻¹; 1445 cm⁻¹; 1367 cm⁻¹; 1286 cm⁻¹; 1162 cm⁻¹; 1116 cm⁻¹; 1065 cm⁻¹; 788 cm⁻¹.
NMR: 250 Mhz 1H-NMR (CDCl₃) (δ ppm): (Imine tautomer) 4.632 (t, H, CH); 4,512 (s br., H, NH); 4,112 (q, 2H, CH2); 4,102 (s, 2H, CH2); 3,705 (q, 2H, CH2); 3,576 (q, 2H, CH2); 3,508 (d, 2H, CH2); 1,266 (m, 9H, CH3)
FAB-MS: 261 [MH⁺], 216, 170

### Example D . 4-(2-chloro-phenyl)-2-(2,2-diethoxyethoxymethyl)-6-methyl-1,4-dihydro-pyridine-3,5-dicarboxylic acid 3-ethyl ester 5-methyl ester (4).

A mixture of 83 g (0.218 mol) 3-(2-chloro-phenyl)-2-[2-(2,2-diethoxy-ethoxy)-acetyl]-acrylic acid ethyl ester (cis and trans isomer) (2) and 25 g (0.218 mol) of methyl-3-aminocrotonate in 800 ml toluene was refluxed in a Dean-Stark water separator for 30 hours. Toluene was evaporated off to give 104 g of the crude product (70 % purity, HPLC) as a dark red oil. The crude product was chromatographed on silica with toluene/ethylacetate 20:1 as eluent. Appropriate fractions were combined to give the product (95-98 % purity, HPLC) as a light yellow glass, which crystallized after several days of standing.
yield: 56.6 g = 53.9 %
mp. 64-67 °C

| Elemental analysis: | | | | |
|---|---|---|---|---|
| Calculated | C 59.8% | H 6.7% | N 2.9% | Cl 7.4% |
| Found | C 59.59% | H 7.14% | N 2.76% | Cl 7.5% |

IR: 3349 cm⁻¹; 2977 cm⁻¹; 2931 cm⁻¹; 1692 cm⁻¹;1646 cm⁻¹; 1611 cm⁻¹; 1482 cm⁻¹; 1208 cm⁻¹; 1163 cm⁻¹; 1100 cm⁻¹; 1060 cm⁻¹; 757 cm⁻¹; (KBr)
NMR: 500 MHz 1H-NMR (CDCl₃) (δ ppm):
7.41 (br. s, 1H, NH); 7.08-7.39 (m, 4H, ArH); 5.42 (s, 1H, CH); 4.774 (d.d., 2H, CH2); 4.684 (t, 1H, CH); 4.04 (q, 2H, CH2); 3.74 (m, 2H, CH2); 3.61 (s, 3H, CH3); 3.58 (s, 4H, CH2); 2.35 (s, 3H ,CH3); 1.26 (d. t, 6H, CH3); 1.176 (d. t, 3H, CH3).
FAB-MS: 482 [MH⁺],481 [M⁺],370 [M⁺-C₆H₄Cl]

### Example D2. 4-(2-chloro-phenyl)-2-(2,2-diethoxyethoxymethyl)-6-methyl-1,4-dihydro-pyridine-3,5-dicarboxylic acid 3-ethyl ester 5-methyl ester (4).

26,13 g (0,1 mol) 3-amino-4-(2,2-diethoxy-ethoxy)-but-2-enoic acid ethyl ester (3) and 23.90 g (0.1 mol) 2-acetyl-3-(2-chloro-phenyl)-acrylic acid methyl ester was dissolved in 200 ml toluene and refluxed in a Dean-Stark water separator for 26 hours. Toluene was evaporated off to give the crude product (88% purity, HPLC) as a yellow oil, which became semicrystalline overnight. The semicrystalline product was stirred vigorously with hexane for a few hours. The resulting crystalline product was filtered off and dried to give 28 g of the pure product. The hexane was evaporated off and the residue was chromatographed on silica with toluene/ethylacetate 20:1 as eluent. Appropriate fractions were combined to give the product (95-98% purity, HPLC) as a light yellow glass, which was stirred with hexane for a few hours. This gave further 8,30 g of pure product after filtering and drying.
Combined yield: 36.3 g = 75.3 %
mp. 64-67 °C

| Elemental analysis: | | | | |
|---|---|---|---|---|
| Calculated | C 59.8% | H 6.7% | N 2.9% | Cl 7.4% |
| Found | C 59.59% | H 7.14% | N 2.76% | Cl 7.5% |

IR: 3349 cm⁻¹; 2977 cm⁻¹; 2931 cm⁻¹; 1692 cm⁻¹; 1646 cm⁻¹; 1611 cm⁻¹; 1482 cm⁻¹; 1208 cm⁻¹; 1163 cm⁻¹; 1100 cm⁻¹; 1060 cm⁻¹; 757 cm⁻¹;
(KBr)
NMR: 500 MHz 1H-NMR (CDCl₃) (δ ppm):
7.41 (br. s, 1H, NH); 7.08-7.39 (m, 4H, ArH) 5.42 (s, 1H, CH); 4.774 (d.d., 2H, CH2); 4.684 (t, 1H, CH); 4.04 (q, 2H, CH2); 3.74 (m, 2H, CH2); 3.61 (s, 3H, CH3); 3.58 (s, 4H, CH2); 2.35 (s, 3H, CH3); 1.26 (d. t, 6H, CH3); 1.176 (d. t, 3H, CH3).
FAB-MS: 482 [MH⁺],481 [M⁺],370 [M⁺-C₆H₄Cl]

### Example E. 1-Benzyl-4-(2-chloro-phenyl)-2-(2,2-diethoxy-ethoxymethyl)-6-methyl-1,4-dihydro-pyridine-3,5-dicarboxylic acid 3-ethyl ester 5-methyl ester (7).

A mixture of 9.6 g (0.025 mol) 3-(2-chlorophenyl)-2-[2-(2,2-diethoxy-ethoxy)-acetyl]-acrylic acid ethyl ester (cis and trans isomer) **(2)** and 5.12 g (0.025 mol) of methyl-3-benzylaminocrotonate in 250 ml toluene was refluxed in a Dean-Stark water separator for 48 hours. The toluene was evaporated off to give 14 g of the crude product (68 % purity) as a dark red oil. The crude product was chromatographed on silica with chloroform as eluent.

Appropriate fractions were combined to give the product (95-98% purity) as a light brown oil.
yield: 7.8 g = 55 %

| Elemental analysis: | | | | |
|---|---|---|---|---|
| Calculated | C 65.1% | H 6.7% | N 2.4% | Cl 6.2% |
| Found | C 64.38% | H 7.06% | N 2.34% | Cl 6.4% |

FAB-MS: 572 [MH⁺], 571 [M⁺]

### Example F. 2,2-Diethoxy-ethanol.

302,6 g (8 mol) of sodium borohydride was added to 2 1 of 1,2-dimethoxyethane (monoglyme) with stirring, after which 704,8 g (4 mol) ethyl diethoxyacetate dissolved in 4 1 of ethanol was added dropwise within 4 hours so that the temperature was kept below 50 °C. The mixture was then heated to reflux for 3 hours. Then 2 1 of ethanol was distilled off, and 4 1 of water was added dropwise while the remaining ethanol and then the 1,2-dimethoxyethane was removed by distillation. During the water addition an abundant precipitate was formed which dissolved towards the end of the addition.

The mixture was cooled on an icebath and 600 g of potassium carbonate was dissolved therein while stirring. The mixture was extracted with 2 1 of diethyl ether and dried with MgSO₄. The diethyl ether was evaporated off and the crude product was distilled in vacuo at 75-76 °C (15 mm Hg).
Yield = 475.8 g = 88.7 %

| Elemental analysis: | | |
|---|---|---|
| Calculated | C 53.7% | H 10.5% |
| Found | C 53.11% | H 10.57% |

IR: 3441 cm⁻¹; 2976 cm⁻¹; 2931 cm⁻¹ ; 2883 cm⁻¹; 1445 cm⁻¹ 1374 cm⁻¹; 1345 cm⁻¹; 1235 cm⁻¹; 1134 cm⁻¹; 1073 cm⁻¹ (Between KBr plates)

### Examples illustrating the process according to the invention.

### Example 1. 2-(2-aminoethoxymethyl)-4-(2-chlorophenyl)-6-methyl-1,4-dihydropyridine-3,5-dicarboxylic acid 3-ethyl ester 5-methyl ester, maleate; Amlodipine maleate.

### A. 4-(2-chloro-phenyl)-2-(2-hydroxyimino-ethoxymethyl)-6-methyl-1,4-dihydro-pyridine-3,5-dicarboxylic acid 3-ethyl ester 5-methyl ester (5).

2,4 g (5 mmol) 4-(2-chloro-phenyl)-2-(2,2-diethoxy-ethoxymethyl)-6-methyl-1,4-dihydro-pyridine-3,5-- dicarboxylic acid 3-ethyl ester 5-methyl ester (4) was dissolved in 75 ml methanol. 20 ml of 0,5 M hydroxylamine hydrochloride in water was added, and the mixture was refluxed for four hours. The methanol was evaporated off, and 75 ml of chloroform was added. The organic phase was washed twice with 75 ml of water and then dried with magnesium sulphate. The chloroform was evaporated off leaving the crude product which was stirred for a few hours with 40 ml of petroleum ether bp. 60-80 °C and 10 - 15 ml of toluene. The precipitate was filtered off and then washed with petroleum ether, giving the product as a white powder.
yield: 1.40 g = 66.2 %
mp. 159-160 °C

| Elemental analysis: | | | | |
|---|---|---|---|---|
| Calculated | C 56.8% | H 5.5% | N 6.6% | Cl 8.4 |
| Found | C 56.8% | H 5.67% | N 6.4% | Cl 8.5% |

IR: 3405 cm⁻¹; 2982 cm⁻¹; 2947 cm⁻¹; 1694 cm⁻¹;1607 cm⁻¹; 1481 cm⁻¹; 1310 cm⁻¹; 1284 cm⁻¹; 1211 cm⁻¹; 1101 cm⁻¹; 758 cm⁻¹; (KBr)
FAB-MS: 423 [MH⁺],422 [M⁺],311 [M⁺-C₆H₄Cl]
NMR: 500 MHz 1H-NMR (CDCl₃) (δ ppm) :
6.96-7.57 (m, 6H, ArH, CH, NH) 5.414 (s, 1H, CH); 4.774 (d.d., 2H, CH2); 4.462 (d, 1H, OH); 4.232 (d. d, 2H, CH2); 4.048 (m, 2H, CH2); 3.618 (s, 3H, CH3); 2.34 (s, 3H, CH3); 1.18 (t, 3H, CH3).

### B. 2-(2-aminoethoxymethyl)-4-(2-chlorophenyl)-6-methyl-1,4-dihydropyridine-3,5-dicarboxylic acid 3-ethyl ester 5-methyl ester, maleate; Amlodipine maleate.

1.00 g (2.4 mmol) 4-(2-chloro-phenyl)-2-(2-hydroxyimino-ethoxymethyl)-6-methyl-1,4-dihydro-pyridine-3,5-dicarboxylic acid 3-ethyl ester 5-methyl ester (5) was dissolved in 20 ml glacial acetic acid and hydrogenated for four hours at atmospheric pressure and room temperature using 0.062 g palladium 10% on carbon as catalyst. The catalyst was filtered off after which the acetic acid was evaporated off. The residue was dissolved in ether and washed successively with 10% sodium bicarbonate solution and water. After drying with MgSO₄ the ether was evaporated off and the residue was dissolved in a small volume of ethanol. To the resulting solution 0.28 g (2.4 mmol) of maleic acid was added with cooling. The maleate salt precipitated after a while and was then filtered off and washed with diethyl ether and dried in vacuo, giving 0.9 g of the product as a fine white powder. By addition of a small amount of ether to the mother liquor further 270 mg of product was obtained.
Combined yield: 1.17 g = 92.9 %
mp. 170-172 °C

| Elemental analysis: | | | | |
|---|---|---|---|---|
| Calculated | C 54.9% | H 5.6% | N 5.3% | Cl 6.8% |
| Found | C 53.86% | H 5.6% | N 5.11% | Cl 6.9% |

IR: 3392 cm⁻¹; 2946 cm⁻¹; 1688 cm⁻¹; 1648 cm⁻¹;1603 cm⁻¹; 1479 cm⁻¹; 1283 cm⁻¹; 1206 cm⁻¹; 1100 cm⁻¹; 759 cm⁻¹; (KBr)
FAB-MS: 409 [MH⁺],408 [M⁺],297 [M⁺-C₆H₄Cl]
NMR: 500 MHz 1H-NMR (D₆-DMSO) (δ ppm) :
8.37 (s, 1H, NH); 7.87 (br. s, 3H, NH); 7.10-7.35 (m, 4H, ArH); 6.06 (s, 2H, CH); 5.31 (s, 1H, CH); 4.66 (d. d., 2H, CH2); 3.97 (q, 2H, CH2); 3.66 (t, 2H, CH2); 3.50 (s, 3H, CH3); 3.09 (t, 2H, CH3); 2.3 (t, 3H, CH3); 1.12 (t, 3H, CH3).

### Example 2. 2-(2-aminoethoxymethyl)-4-(2-chlorophenyl)-6-methyl-1,4-dihydropyridine-3,5-dicarboxylic acid 3-ethyl ester 5-methyl ester, maleate; Amlodipine maleate.

0.5 g (1.2 mmol) 4-(2-chloro-phenyl)-2-(2-hydroxyimino-ethoxymethyl)-6-methyl-1,4-dihydro-pyridine--3,5-dicarboxylic acid 3-ethyl ester 5-methyl ester (5) and 0.56 g (2.4 mmol) nickel chloride hydrate was dissolved in 35 ml methanol and cooled to -30 °C. 0.454 g (0.012 mol) sodium borohydride was added in small portions over 30 minutes whereafter the mixture was stirred for 1 hour at room temperature. The mixture was evaporated to dryness and 20 ml of 6N hydrochloric acid was added with stirring. The mixture was filtered and then made alkaline with conc. ammonium hydroxide (pH > 8.5). The filtrate was extracted twice with dichloromethane, and then dried with MgSO₄. The organic phase was evaporated to give 0.274 g (56.7 %) of the crude base. The product was dissolved in a small amount of ethanol. To the resulting solution 0.08 g (0.7 mmol) maleic acid was added with cooling. After a while the maleate salt precipitated and was then filtered off and washed with diethyl ether and dried in vacuo, giving the product as a fine white powder.
yield: 0.21 g = 33.4 %
mp. 170-172 °C

| Elemental analysis: | | | | |
|---|---|---|---|---|
| Calculated | C 54.9% | H 5.6% | N 5.3% | Cl 6.8% |
| Found | C 54.94% | H 5.65% | N 5.23% | Cl 7.05% |

IR: 3392 cm⁻¹; 2946 cm⁻¹; 1688 cm⁻¹; 1648 cm⁻¹; 1603 cm⁻¹; 1479 cm⁻¹; 1283 cm⁻¹; 1206 cm⁻¹; 1100 cm⁻¹; 759 cm⁻¹; (KBr)
FAB-MS: 409 [MH⁺],408 [M⁺],297 [M⁺-C₆H₄Cl]

### Example 3. 4-(2-chloro-phenyl)-2-(2-benzyloxy-imino-ethoxymethyl)-6-methyl-1,4-dihydro-pyridine-3,5-dicarboxylic acid 3-ethyl ester 5-methyl ester (8).

2,4 g (5 mmol) 4-(2-chloro-phenyl)-2-(2,2-diethoxy-ethoxymethyl)-methyl-1,4-dihydro-pyridine-3,5-dicarboxylic acid 3-ethyl ester 5-methyl ester (4) was dissolved in 75 ml methanol. 20 ml of 0,5 M O-benzylhydroxylamine hydrochloride in water was added, and the mixture was refluxed for four hours. The methanol was evaporated off, and 75 ml of dichloromethane was added. The organic phase was washed twice with 75 ml of water and then dried with magnesium sulphate. Dichloromethane was evaporated off leaving the product as an oil. The crude product was chromatographed on silica with chloroform as eluent. Appropriate fractions were combined to give the product as a brown oil.
yield: 2.3 g = 90 %

| Elemental analysis: | | | | |
|---|---|---|---|---|
| Calculated | C 63.2% | H 5.7% | N 5.5% | Cl 6.9% |
| Found | C 62.5% | H 5.8% | N 5.2 | Cl 6.6% |

FAB-MS: 513 [MH⁺],512 [M⁺],401 [M⁺-C₆H₄Cl]

In the preceding the invention has been described by means of specific examples of preferred embodiments. However, it will be appreciated by a person skilled in the art that various modifications can be made without deviating from the spirit and scope of the invention.

## Claims

1. A process for the preparation of 1,4-dihydropyridines of the general formula I 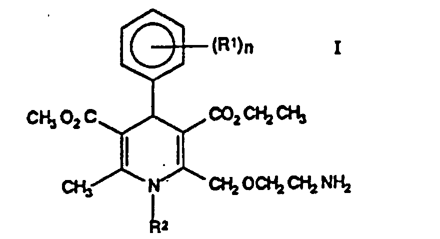 wherein
R¹ each, independently, represents H, Cl or CF₃,
R² represents H, C₁-C₅ alkyl, C₃-C₆ cycloalkyl or aralkyl, and
n is 1 or 2,
or acid addition salts thereof,
**characterized in** reacting an acetal of the general formula II 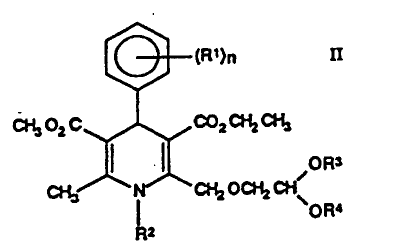 obtained by a Hantzsch synthesis carried out using a compound containing a group of the formula 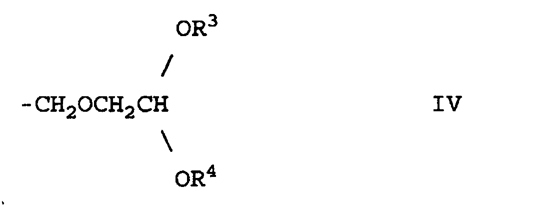 as one of the reactants in the Hantzsch condensation, in which formulae II and IV
R³ and R⁴, which may be the same or different, represent C₁-C₅ alkyl, C₃-C₆ cycloalkyl, aralkyl or together represent -(CH₂)ₘ-, wherein
m is 2 or 3, and
R¹, R² and n have the same meanings as defined above,
with
R⁵ONH₂
or an acid addition salt thereof, wherein
R⁵ represents H, C₁-C₅ alkyl, C₃-C₆ cycloalkyl or aralkyl,
so as to provide an oxime of the general formula III 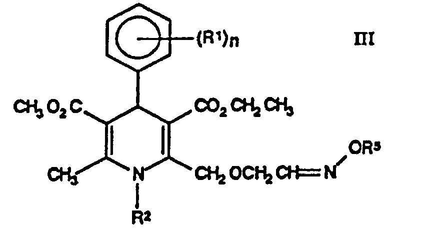 wherein
R¹, R², R⁵ and n have the same meanings as defined above, and
reducing the formed oxime of formula III so as to provide a 1,4-dihydropyridine of formula I, and, if desired, converting a compound of formula I obtained as the free base into a pharmaceutically acceptable acid addition salt thereof or vice versa.

2. A process according to claim 1, wherein the reduction of the oxime of formula III is carried out by catalytical hydrogenation.

3. A process according to claim 2, wherein the catalytical hydrogenation is carried out using a platinum, palladium or Raney nickel catalyst.

4. A process according to any of claims 1 - 3, wherein the reduction is carried out under acidic conditions.

5. A process according to any of claims 1 - 4, wherein the reduction is carried out by catalytical hydrogenation in acetic acid using palladium-on-carbon as a catalyst.

6. A process according to claim 1, wherein the reduction of the oxime of formula III is carried out using sodium borohydride/nickel chloride hydrate as reduction agent.

7. A process according to any of claims 1 - 6, wherein the Hantzsch synthesis, or at least one step thereof, is carried out in a solvent being capable of forming an azeotrope with water, particularly toluene, benzene or xylene.

8. A process according to any of claims 1 - 7, wherein the Hantzsch synthesis, or at least one step thereof, is carried out under azeotropic removal of water of reaction.

9. A process according to any of claims 1 - 8, wherein n is 1, R¹ is chloro in the 2-position of the phenyl ring, and R² is H.

## Patentansprüche

1. Verfahren zur Herstellung von 1,4-Dihydropyridinen der allgemeinen Formel I 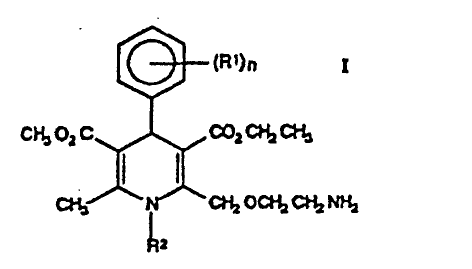 worin
R¹ jeweils unabhängig H, Cl oder CF₃ darstellt,
R² H, C₁-C₅-Alkyl, C₃-C₆-Cycloalkyl oder Aralkyl darstellt,
n gleich 1 oder 2 ist,
oder von Säureadditionssalzen davon,
**gekennzeichnet durch** die Umsetzung eines Acetals der allgemeinen Formel II 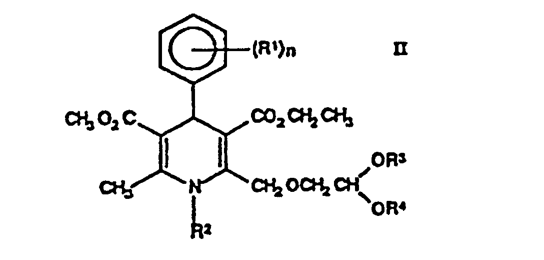 das **durch** eine Hantzsch-Synthese erhalten wurde, die unter Verwendung einer eine Gruppe mit der Formel 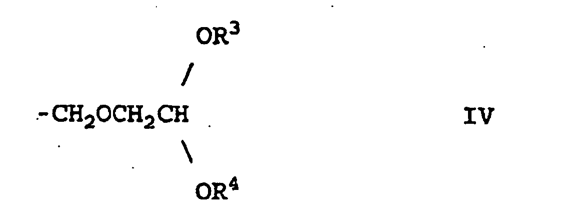 enthaltenden Verbindung als eines der Reaktanten in der Hantzsch-Kondensation durchgeführt wurde, wobei in den Formeln II und IV
R³ und R⁴, die gleich oder verschieden sein können, C₁-C₅-Alkyl, C₃-C₆-Cycloalkyl, Aralkyl oder zusammen - (CH₂)ₘ- darstellen, wobei m 2 oder 3 ist, und R¹, R² und n die oben angegebene Bedeutung haben,
mit
R⁵ONH₂
oder einem Säureadditionssalz davon, wobei R⁵ H, C₁-C₅-Alkyl, C₃-C₆-Cycloalkyl oder Aralkyl darstellt,
um ein Oxim der allgemeinen Formel III zu erhalten, 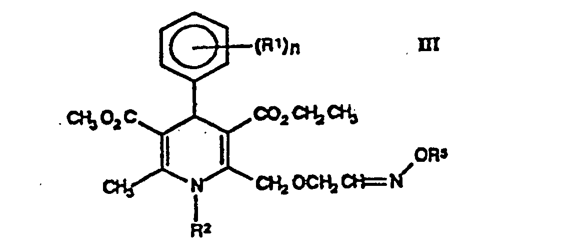 worin R¹, R², R⁵ und n die oben angegebene Bedeutung haben,
und Reduktion des gebildeten Oxims der Formel III, um ein 1,4-Dihydropyridin der Formel I zu erhalten, und, falls das gewünscht wird, Umwandeln einer als freie Base erhaltenen Verbindung der Formel I in ein pharmazeutisch unbedenkliches Säureadditionssalz oder umgekehrt.

2. Verfahren nach Anspruch 1, wobei die Reduktion des Oxims der Formel III durch katalytische Hydrierung erfolgt.

3. Verfahren nach Anspruch 2, wobei die katalytische Hydrierung unter Verwendung eines Platin-, Palladium- oder Raney-Nickel-Katalysators durchgeführt wird.

4. Verfahren nach einem der Ansprüche 1 bis 3, wobei die Reduktion unter sauren Bedingungen durchgeführt wird.

5. Verfahren nach einem der Ansprüche 1 bis 4, wobei die Reduktion durch katalytische Hydrierung in Essigsäure unter Verwendung von Palladium auf Kohle als Katalysator durchgeführt wird.

6. Verfahren nach Anspruch 1, wobei die Reduktion des Oxims der Formel III unter Verwendung von Natriumborhydrid/Nicketchtorid-Hydrat als Reduktionsmittel durchgeführt wird.

7. Verfahren nach einem der Ansprüche 1 bis 6, wobei die Hantzsch-Synthese oder mindestens ein Schritt davon in einem Lösungsmittel durchgeführt wird, das mit Wasser ein Azeotrop bilden kann. insbesondere Toluol, Benzol oder Xylol.

8. Verfahren nach einem der Ansprüche 1 bis 7, wobei die Hantzsch-Synthese oder mindestens ein Schritt davon unter azeotroper Entfernung von Reaktionswasser durchgeführt wird.

9. Verfahren nach einem der Ansprüche 1 bis 8, wobei n gleich 1, R¹ Chlor in der Position 2 des Phenylrings und R² gleich H ist.

## Revendications

1. Procédé de préparation de 1,4-dihydropyridines de la formule générale I 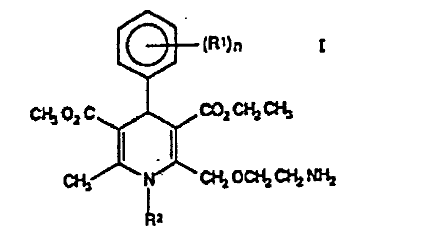 où
R¹ chacun indépendamment représente H, Cl ou CF₃,
R² représente H, C₁-C₅ alkyle, C₃-C₆ cycloalkyle ou aralkyle, et
n est 1 ou 2,
ou des sels d'addition d'acide de ceux-ci,
**caractérisé** en réagissant un acétal de la formule générale II 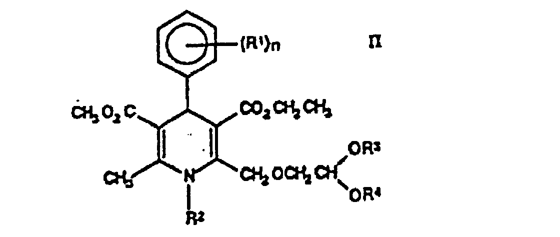 obtenu par une synthèse Hantzsch effectuée à l'usage d'un composé contenant un groupement de la formule 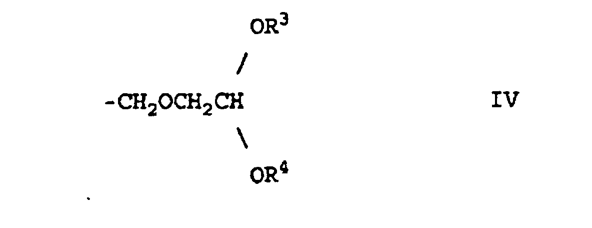 en tant que l'un des réactants dans la condensation Hantzsch, formules II et IV dans lesquelles
R³ et R⁴, qui peuvent être identiques ou différents, représentent C₁-C₅ alkyle, C₃-C₆ cycloalkyle, aralkyle ou ensemble représentent -(CH₂)ₘ-, où
m est 2 ou 3, et
R¹, R² et n ont les mêmes sens que ceux définis plus hauts,
avec
R⁵ONH₂
ou un sel d'addition d'acide de celui-ci, où
R⁵ représente H, C₁-C₅ alkyle, C₃-C₆ cycloalkyle ou aralkyle,
afin d'obtenir une oxime de la formule générale III 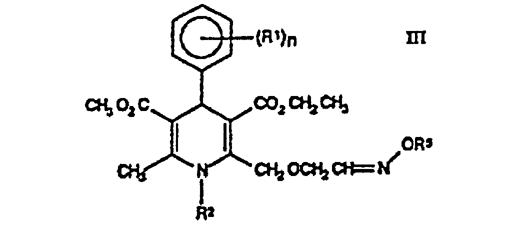 ou
R¹, R², R⁵ et n ont les mêmes sens que ceux définis plus haut, et
réduisant l'oxime formée de la formule III afin d'obtenir un 1,4-dihydropyridine de la formule I, et, si désiré, convertir un composé de la formule I obtenu comme la base libre en un sel d'addition d'acide pharmaceutiquement acceptable de celle-la ou vice versa.

2. Procédé selon la revendication 1, où la réduction de l'oxime de la formule III est effectuée par hydrogénation catalytique.

3. Procédé selon la revendication 2, où l'hydrogénation catalytique est effectuée à l'usage d'un catalyseur de platine, de palladium ou de nickel de Raney.

4. Procédé selon l'une quelconque des revendications 1-3, où la réduction est effectuée sous des conditions acides.

5. Procédé selon l'une quelconque des revendications 1-4, où la réduction est effectuée par hydrogénation catalytique en acide acétique à l'usage de palladium-sur-carbone en tant que catalyseur.

6. Procédé selon la revendication 1, où la réduction de l'oxime de la formule III est effectuée à l'usage de borohydrure de sodium/hydrate de chlorure de nickel en tant qu'agent réducteur.

7. Procédé selon l'une quelconque des revendications 1-6, où la synthèse Hantzsch, ou à moins une étape de celle-là, est effectuée dans un solvant susceptible de créer un azéotrope avec de l'eau, particulièrement toluène, benzène ou xylène.

8. Procédé selon l'une quelconque des revendications 1-7, où la synthèse Hantzsch, ou à moins une étape de celle-là, est effectuée sous élimination azéotrope d'eau de réaction.

9. Procédé selon l'une quelconque des revendications 1-8, où n est 1, R¹ est chloro dans la 2-position de l'anneau phényle, et R² est H.
